# EUROPÄISCHE PATENTSCHRIFT

(11) **EP 0 823 850 B1**
(45) Veröffentlichungstag und Bekanntmachung des Hinweises auf die Patenterteilung: **08.12.1999**
(21) Anmeldenummer: 96914174.6
(22) Anmeldetag: 03.05.1996
(51) Int. Cl.: A61M 16/10

(54) **VORRICHTUNG ZUR ERZEUGUNG GESUNDHEITSVERTRÄGLICHER ATEMLUFT IN NASALEN ÜBERDRUCK-BEATMUNGSGERÄTEN**
DEVICE FOR PRODUCING RESPIRATORY AIR WHICH IS HARMLESS TO HEALTH IN POSITIVE PRESSURE NASAL BREATHING APPARATUS
DISPOSITIF PERMETTANT DE PRODUIRE DE L'AIR NON PREJUDICIABLE A LA SANTE DANS DES APPAREILS A SURPRESSION POUR RESPIRATION NASALE

(30) Priorität: 03.05.1995 DE 19515739
(43) Veröffentlichungstag der Anmeldung: 18.02.1998
(73) Patentinhaber: Krohn, Holger, 97816 Lohr (DE)
(72) Erfinder: Krohn, Holger, 97816 Lohr (DE)
(74) Vertreter: Wagner, Karl H., Dipl.-Ing.
(86) Internationale Anmeldenummer: EP9601842
(87) Internationale Veröffentlichungsnummer: WO9634644

(56) Entgegenhaltungen:
- WO-A-93/20874
- DE-A- 4 111 138
- DE-C- 634 919
- GB-A- 161 969
- PATENT ABSTRACTS OF JAPAN vol. 014, no. 305 (M-0992), 29.Juni 1990 & JP,A,02 097881 (SNOW BRAND MILK PROD CO LTD), 10.April 1990,

## Beschreibung

Nasale Überdruck-Beatmungsgeräte werden zur Vermeidung sogenannter obstruktiver Schlafapnoen eingesetzt.

Bei obstruktiver Schlafapnoen handelt es sich um ein im Schlaf wiederholt auftretendes Zusammenfallen der Atemwege im Schlundbereich, das zu Atemstillständen führt und bei häufigem Auftreten nachhaltige Gesundheitsschäden durch Schlafmangel und Sauerstoffmangel verursacht.

Bei den unter dem Begriff nCPAP (nasal Continuous Positive Airway Pressure) bekannten Überdruck - Beatmungsgeräten erzeugt ein Gebläse einen konstanten Druck, der über einen Schlauch und eine Nasenmaske in die Atemwege geleitet wird. Der positive Druck in den Atemwegen verhindert den Verschluß der Schlundmuskulatur und beseitigt so die Atemstillstände.

Zur Abführung der ausgeatmeten verbrauchten Luft wird an der Atemmaske ein kontinuierlicher Abluftstrom über feine Austrittsöffnungen abgeblasen.

Die Abluft-Austrittsöffnungen müssen eng ausgeführt sein, damit eine Drosselwirkung entsteht und der Überdruck nicht zusammenbricht, sondern nur eine bestimmte Abluftmenge austritt.

Um die verbrauchte Luft vollständig abzuführen muß die Abluftmenge mindestens so groß sein wie die ausgeatmete Luftmenge.

Eine gesundheitsschädliche Wirkung ergibt sich bei der Überdruckbeatmung dadurch, daß die Luft durch die Verdichtung in dem Gebläse erwärmt wird, und die relative Luftfeuchte infolgedessen naturgesetzmäßig abnimmt. Die infolge des Abluftstromes ständig strömende trockene Luft, trocknet die Schleimhäute der Atemwege aus und verursacht dadurch ständige, mit einem Schnupfen vergleichbare Beschwerden.

Um diese Probleme zu vermeiden, ist es bekannt, zwischen dem Gebläse und der Atemmaske Luftbefeuchter einzusetzen.

In den Anfängen der nasalen Überdruck - Beatmung verwendete man für diesen Zweck aufwendige geregelte Atemlufbefeuchter, wie sie in der Intensivmedizin Anwendung finden.

Zwischenzeitlich hat sich bei zehntausenden Schlafapnoe - Betroffenen eine häusliche Überdruck - Beatmung im Schlaf als lebensnotwendig erwiesen.

Für diesen Zweck sind die aufwendigen Atemluftbefeuchter der Intensivmedizin zu kompliziert und zu teuer.

Es wurde deshalb bekannt, sogenannte Kalt - Luftanfeuchter zu verwenden. Dabei durchströmt der vom Gebläse kommende Luftstrom ein geschlossenes Gehäuse, in dem Wasser mittels einer Pumpe versprudelt wird.

Da sich bei der Anwendung jedoch zeigte, daß mit den Kalt - Luftbefeuchtern die Austrocknung der Schleimhäute in den Atemwegen nicht hinreichend vermieden werden kann, werden derzeit überwiegend sogenannte Warm - Luftbefeuchter eingesetzt, wie in der Bedienungsanleitung des "Sullivan Atemgasanfeuchters Modell HC 100" der PRIESS med. Technik, Karstrasse 17a, 41068 Mönchengladbach beschrieben.

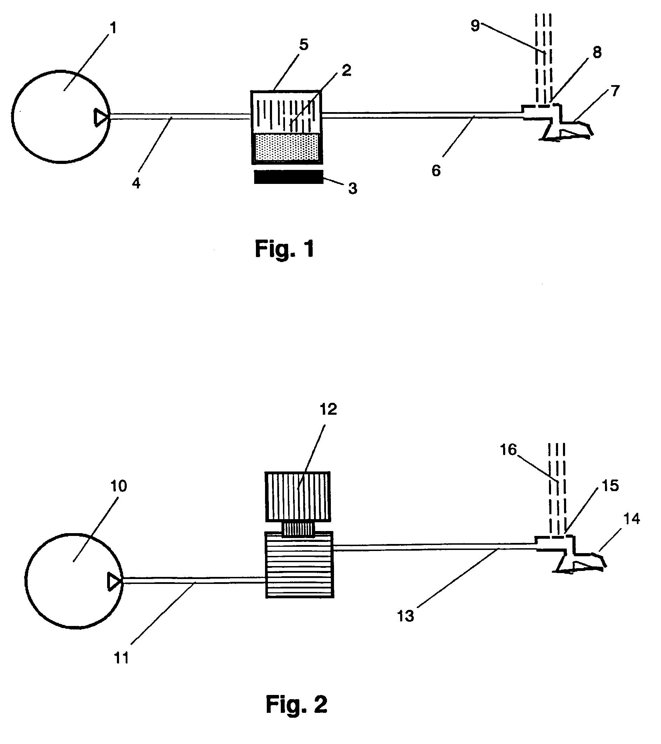

In Fig. 1 der Zeichnung ist dieser Stand der Technik schematisch dargestellt.

Das Gebläse 1 erzeugt einen Luftstrom mit einem Überdruck von ca. 3 bis 18 Millibar. Hierbei erwärmt sich der Luftstrom um ca. 1 bis 2° C über die Umgebungstemperatur.

Über einen Schlauch 4 wird der Luftstrom vom Gebläse 1 zum Luftbefeuchter 5 geleitet, und von dort wieder über einen Schlauch 6 zur Nasenmaske 7 und den feinen Austrittsöffnungen 8 für den Abluftstrom 9 weitergeleitet. Im Verdunstungsgefäß 2 des Luftbefeuchters wird das eingefüllte Wasser durch eine elektrische Beheizung 3 verdunstet. Der durchströmende Luftstrom nimmt im Verdunstungsgefäß 2 diesen Wasserdampf auf, und wird dadurch gleichzeitig auf ca. 4 bis 6° C über die Umgebungstemperatur erwärmt.

Mit solchen Warm - Luftbefeuchtern wird eine hinreichende Befeuchtung der Atemluft erzielt, um das Austrocknen der Schleimhäute zu vermeiden.

Die Warmbefeuchtung der Atemluft verursacht jedoch die nachstehenden ernsten Probleme:

Durch die Warmbefeuchtung wird die, durch das Gebläse ohnehin erwärmte Luft, noch weiter erwärmt. Das Einatmen dieser warmen und feuchten Luft verursacht subjektive Atemnot und verursacht dadurch häufig Schlafunterbrechungen und nachfolgend erhebliche Einschlafprobleme.

Da die Temperatur der warmbefeuchteten Luft beim Austritt aus dem Verdampfungsgefäß höher ist als die umgebende Raumtemperatur, fällt in dem Beatmungsschlauch 6 und in der Nasenmaske 7 Kondenswasser an.

Um die Bildung gesundheitsschädlicher Kulturen infolge dieser Feuchtigkeit zu vermeiden, muß der Schlauch 6 täglich gründlich ausgewaschen und getrocknet werden.

Ein ernstes Problem entsteht außerdem an den feinen Austrittsöffnungen 8 für die Abluft 9. Da die Abluft 9 beim Austritt expandiert und infolgedessen adiabatisch abkühlt, fällt weiteres Kondenswasser aus der feuchtigkeitsgesättigten Luft in den feinen Luftaustrittsöffnungen 8 aus, und verstopft diese teilweise. Dadurch wird die ausgeatmete Luft nicht mehr hinreichend abgeführt. Die Folge sind Schlaf- und Gesundheitsstörungen durch Sauerstoffmangel.

Zum Stand der Technik sei ferner auf die DE-C-6 34 919 und die WO-A-9 320 874 hingewiesen, die eine Atmungskammer mit Kühlluft bzw. eine Vorrichtung zur Zerstörung von Mikroorganismen betreffen.

Die Aufgabe der Erfindung besteht darin, eine Vorrichtung zur Erzeugung von Atemluft zur häuslichen Überdruck - CPAP (Continuous Positive Airway Pressure) Beatmung von Personen mit obstruktiven Schlafanoesyndrom derart vorzusehen, daß bei der Herstellung einer gesundheitsverträglichen Luftfeuchte der Atemluft das Auftreten von Kondenswasser vermieden wird.

Zur Lösung dieser Aufgabe sind die im Anspruch 1 genannten Maßnahmen vorgesehen. Bevorzugte Weiterbildungen der Erfindung ergeben sich aus den Unteransprüchen.

Bevor auf weitere Vorteile der Erfindung eingegangen wird, sei im Hinblick auf den oben genannten Stand der Technik bemerkt, daß bei der erfindungsgemäßen Vorrichtung ein unter Überdruck stehender Luftstrom erzeugt wird, der vor seiner Zuführung zu einer Nasenmaske und Austrittsöffnungen einer Behandlung, im Falle des Standes der Technik einer Befeuchtungsbehandlung unterworfen wird.

Die ausreichende Luftfeuchte wird mit einfachen Mitteln erzielt, ohne die Mängel der bekannten Überdruckbeatmungsgeräte, die erhöhte Temperatur der Atemluft, die Gefahr der Bildung von gesundheitsschädlichen Bakterien und Sauerstoffmangel infolge der Verstopfung der Luftaustrittsöffnungen durch Kondensat in Kauf nehmen zu müssen.

Die relative Luftfeuchte ist bei unveränderter absoluter Luftfeuchte bekanntlich durch die Lufttemperatur bestimmt. Diese Naturgesetzmäßigkeit verursacht die schädliche Absenkung der relativen Luftfeuchte durch Erwärmung um ca. 1 bis 2° C bei der Erzeugung des Überdrucks in dem Gebläse des Überdruck - Beatmungsgerätes.

Wird nun die vom Gebläse gelieferte Luft mit geeigneten Mitteln um ca. 5° C abgekühlt, so ist die relative Luftfeuchte höher als vor der Verdichtung.

Da die Temperatur der gekühlten Luft dann ca. 3° C niedriger ist als die Umgebungstemperatur, fällt trotz hoher relativer Luftfeuchte weder im Atemschlauch noch in den Abluftschlitzen der Nasenmaske Kondenswasser an.

Der Atemschlauch muß nicht mehr täglich ausgewaschen und getrocknet werden. Die Abführung der verbrauchten Atemluft wird nicht durch Kondenswasser in den Abluftöffnungen behindert. Sauerstoffmangel infolge unzureichender Abluftabfuhr ist damit vermieden.

Die gekühlte Atemluft wird subjektiv als frisch empfunden, und ermöglicht einen erholsamen Schlaf.

Außerdem entzieht der erfindungsgemäß gekühlte Luftstrom den Schleimhäuten naturgesetzmäßig weniger Feuchtigkeit als ein Luftstrom gleicher relativer Luftfeuchte aber höherer Temperatur bei den bekannten Verfahren.

Die Kühlung der Atemluft erfolgt erfindungsgemäß mit einem Wärmetauscher, bei dem ein Kühlprofil in einem geschlossenen Gehäuse von dem vom Gebläse geliefertem Luftstrom durchströmt wird.

Die aus dem Luftstrom von max. 30 Liter pro Minute abzuführende Wärmemenge beträgt bei 5° C Temperaturabsenkung nur 3,25 Watt, und kann erfindungsgemäß mit marktgängigen Peltierelementen abgeführt werden.

Die Kaltseite des Peltierelementes wird thermisch mit dem inneren Kühlprofil des Wärmetauschers verbunden. Die Wärme von der heißen Seite des Peltierelementes kann mittels eines belüfteten äußeren Kühlprofils problemlos an die Umgebungsluft abgeführt werden.

Ausgehend vom Stand der Technik gemäß Fig. 1, wird ein Ausführungsbeispiel der Erfindung wird nachfolgend anhand der Figuren 2 - 4 der Zeichnung näher erläutert. In der Zeichnung zeigt:
- Fig. 1:: den Stand der Technik
- Fig. 2:: den schematischen Aufbau einer nasalen Überdruckbeatmungsvorrichtung
- Fig. 3:: den schematischen Aufbau des Wärmetauschers und
- Fig. 4:: eine Ansicht in Richtung A der Fig. 3.

Nach Fig. 2 wird von dem Gebläse 10 ein Luftstrom je nach Einstellung mit einem Überdruck zwischen 3 und 18 Millibar erzeugt, und in den Schlauch 11 zum Wärmetauscher 12 weitergeleitet. Durch die Verdichtung ist der Luftstrom gegenüber der Umgebungstemperatur je nach Überdruck zwischen 1 bis 2° C erwärmt und die relative Luftfeuchte dadurch gegenüber dem Ansaugzustand naturgesetzmäßig reduziert. Im Wärmetauscher 12 wird der Luftstrom auf ca. 3° C unter die Umgebungstemperatur abgekühlt. Dadurch erhöht sich die relative Luftfeuchte des Luftstromes um ca. 20 % gegenüber dem Ansaugzustand. Über den Schlauch 13 wird der Luftstrom der Nasenmaske 14 und den feinen Austrittsöffnungen 15 für den Abluftstrom 16 weitergeleitet.

Aufgrund der höheren Umgebungstemperatur fällt weder im Schlauch 13 noch in den feinen Austrittsöffnungen 15 Kondenswasser aus dem Luftstrom an. Die feinen Austrittsöffnungen 15 für den Abluftstrom 16 bleiben frei, so daß die Atemluft sauerstoffreich und durch die Abkühlung bekömmlich ist.

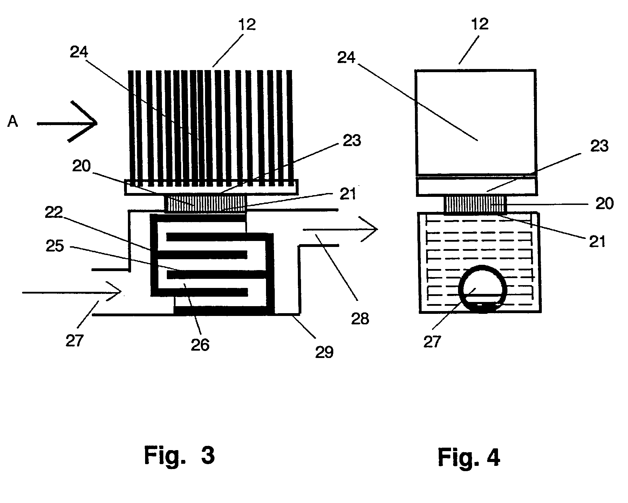

Der Wärmetauscher 12 nach den Figuren 3 und 4 besteht im wesentlichen aus einem Peltierelement 20, dessen kalte Seite 21 mit den Kühlprofilen 22 und 25 aus Metall hoher Wärmeleitfähigkeit, beispielsweise Aluminium, thermisch verbunden ist, und dessen heiße Seite 23 mit einem weiteren Kühlprofil 24 aus Metall mit hoher thermischer Leitfähigkeit verbunden ist.

Über das Kühlprofil 24 wird die durch das Peltierelement 20 elektrisch von der kalten Seite 21 zur heißen Seite 23 gepumpte Wärme aus dem Luftstrom, und die elektrische Verlustleistung des Peltierelementes 20 an die Umgebungsluft abgeführt.

Die beiden mit der kalten Seite 21 des Peltierelements 20 thermisch verbundenen E - förmigen Kühlprofile 22 und 25 bilden zwischen dem Lufteintritt 27 und dem Luftaustritt 28 einen meanderförmigen Kühlkanal 26 für den vom Gebläse 10 gelieferten Luftstrom.

Der Lufteintritt 27 und der Luftaustritt 28 des Gehäuses 29 ist zum Anschluß der Schläuche rohrförmig ausgebildet. Der Lufteintritt 27 befindet sich an der dem Peltierelement 20 entferntesten Stelle des Kühlkanals 26, und der Austritt 28 an der dem Peltierelement nahesten Stelle. Dadurch ergibt bei der stetigen Abkühlung des Luftstromes in dem meanderförmigen Kühlkanal 26 ein nahezu gleichbleibendes Temperaturgefälle zu den Kühlprofilen 22 und 25, und dadurch eine besonders günstige Kühlwirkung des Wärmetauschers.

Selbstverständlich sind auch andere Ausbildungen der Kühlprofile möglich. Insbesondere können zur Vergrößerung der Kühlfläche die Profile durch weitere Schenkel vergrößert werden.

Zusammenfassend kann man sagen, daß die Erfindung ein nasales Überdruck-Beatmungsgerät betrifft, bei dem dem zu behandelnden Menschen ein behandelter einen Überdruck aufweisender Luftstrom zugeführt wird, wobei eine erste Vorrichtung 10 zur Erzeugung eines ersten Luftstromes dient, der eine bestimmte erste Temperatur und eine erste relative Luftfeuchtigkeit aufweist, und wobei eine zweite Vorrichtung 12 den ersten Luftstroms derart verarbeitet, daß ein zweiter dem Menschen ganz oder teilweise zuzuführender Luftstrom erzeugt wird, dessen Temperatur niedriger und dessen relative Luftfeuchtigkeit höher ist als beim ersten Luftstrom.

Die erfindungsgemäße CPAP Beatmungsvorrichtung verwendet ein Gebläse 10, welches die angesaugte Umgebungsluft erwärmt, so daß der erste Luftstrom die bestimmte erste Temperatur besitzt. Die zweite Vorrichtung ist vorzugsweise ein Wärmetauscher, der vorzugsweise nach dem Peltierprinzip arbeitet.

Gemäß einer Weiterbildung ist die zweite Vorrichtung ein einziges Peltierelement, welches vorzugsweise auf Halbleiterbasis ausgebildet ist und eine flache Gestalt von etwa 4 x 4 cm Länge sowie eine Dicke von 5 bis 6 mm besitzt.

Das nasale Überdruck-Beatmungsgerät sieht vorzugsweise vor, daß die durch die Luftstromerzeugungsvorrichtung bzw. das Gebläse 10 um ca. 1 bis 2° C erwärmte Umgebungsluft des ersten Luftstroms mittels des Peltierelements um ungefähr 3 bis 6° C, vorzugsweise 5° C abgekühlt wird, so daß der aus dem Peltierelement austretende zweite Luftstrom um 2 bis 4° C, vorzugsweise ca. 3° C kälter ist als die angesaugte Umgebungsluft, wobei die relative Luftfeuchte des aus dem Peltierelement austretenden zweiten Luftstroms ca. 30 bis 40 % höher liegt als die relative Luftfeuchtigkeit der Ansaugluft, wobei der aus dem Peltierelement austretende zweite Luftstrom sich auf dem Weg zu einer Atemmaske allenfalls erwärmt und dadurch eine etwas verringerte relative Luftfeuchtigkeit aufweist, wobei aber kein Kondensat ausfällt, so daß auch die hinter einer mit dem zweiten Luftstrom ganz oder teilweise versorgten Atemmaske vorhandenen feinen Austrittsöffnungen 8 oder Schlitze für den Abluftstrom sich nicht verstopfen, da kein Kondensat mehr ausfällt.

Vorzugsweise ist das Peltierelement 20 auf seiner heißen Seite 23 mit dem Kühlprofil 24 verbunden, und die kalte Seite 21 weist Kühlprofile derart auf, daß der Kühlkanal 26 für den Durchfluß der Luft gebildet wird. Der Kühlkanal 26 ist wie erwähnt vorzugsweise meanderförmig und wird durch zwei ineinandergreifende Kühlprofile 22, 25 gebildet.

Das erfindungsgemäße nasale Überdruck-Beatmungsgerät unter Verwendung eines Wärmetauschers, insbesondere eines Peltierelements, weist eine den Überdruck der Atemluft erzeugende Vorrichtung 10 auf, die über Schlauchverbindungen 11, 13 mit der Maske 14 in Verbindung steht, die feine Austrittsöffnungen 15 für den Abluftstrom ausweist, wobei in die Verbindung zwischen den Überdruckerzeugungsvorrichtung 10 und der Maske 14 ein Wärmetauscher eingefügt ist.

Es ist auch möglich, das nasale Überdruck-Beatmungsgerät unter Verwendung eines Wärmetauschers, insbesondere eines Peltierelements, derart vorzusehen, daß eine den Überdruck der Atemluft erzeugende Vorrichtung 10 vorgesehen ist, die über Schlauchverbindungen 11, 13 mit einer Maske 14 in Verbindung steht, die feine Austrittsöffnungen 15 für den Abluftstrom ausweist, und wobei das Peltierelement vor der Überdruckerzeugungsvorrichtung 10 angeordnet ist. Ferner kann das nasale Überdruck-Beatmungsgerät unter Verwendung eines Wärmetauschers, insbesondere eines Peltierelements, so ausgebildet sein, daß die den Überdruck der Atemluft erzeugenden Vorrichtung 10, die über Schlauchverbindungen 11, 13 mit der Maske 14 in Verbindung steht, die feine Austrittsöffnungen 15 für den Abluftstrom ausweist, ein Peltierelement vorgeschaltet und ein weiteres Peltierelement nachgeschaltet ist.

## Patentansprüche

1. CPAP (Continuous Positiv Air Pressure) Beatmungsvorrichtung zur häuslichen Überdruck-Beatmung von Personen mit obstruktivem Schlafapnoe Syndrom, mit einem den Überdruck erzeugenden Gebläse (10) und einem in den Luftstrom des Gebläses (10) eingefügten Wärmetauscher (12), der den vom Gebläse (10) gelieferten Luftstrom unter die Umgebungstemperatur abkühlt, bevor er einer Nasenmaske (14) und Austrifföffnungen (15) zugeführt wird.

2. CPAP (Continuous Positiv Air Pressure) Beatmungsvorrichtung nach Anspruch 1, dadurch gekennzeichnet, daß in dem Wärmetauscher (12) ein Peltierelement zur Kühlung vorgesehen ist.

3. CPAP (Continuous Positiv Air Pressure) Beatmungsvorrichtung nach Anspruch 1 oder 2, dadurch gekennzeichnet, daß der Wärmetauscher (12) erste und zweite Kühlprofile (22, 25; 24) aufweist.

4. CPAP (Continuous Positiv Air Pressure) Beatmungsvorrichtung nach Anspruch 3, dadurch gekennzeichnet, daß die kalte Seite (21) des Peltierelementes (20) mit ersten Kühlprofilen (22, 25) und die heiße Seite (23) mit dem zweiten Kühlprofil (24) verbunden ist.

5. CPAP (Continuous Positiv Air Pressure) Beatmungsvorrichtung nach Anspruch 3, dadurch gekennzeichnet, daß die ersten Kühlprofile (22, 25) E-förmig ausgebildet sind und zwischen einem Lufteintritt (27) und einem Luftaustritt (28) einen meanderförmigen Kühlkanal für den vom Gebläse (10) gelieferten Luftstrom bilden.

6. CPAP (Continuous Positiv Air Pressure) Beatmungsvorrichtung nach einem der vorhergehenden Ansprüche, dadurch gekennzeichnet, daß der gelieferte Luftstrom um 2 bis 4°C unter die Umgebungstemperatur abgekühlt wird.

7. CPAP (Continuous Positiv Air Pressure) Beatmungsvorrichtung nach einem der vorhergehenden Ansprüche, dadurch gekennzeichnet, daß der gelieferte Luftstrom um 3°C unter die Umgebungstemperatur abgekühlt wird.

## Claims

1. A CPAP (Continuous Positive Air Pressure) respiratory apparatus for home positive pressure respiration of persons with obstructive sleep apnea syndrome comprising a fan (10) for creating the positive pressure and a heat exchanger (12) inserted into the air stream of the fan (10), said heat exchanger (12) cooling the air stream supplied by the fan (10) below the ambient temperature before it is supplied to a nose mask (14) and exit openings (15).

2. The CPAP (Continuous Positive Air Pressure) respiratory apparatus of claim 1 characterized in that a Peltier element is provided for cooling in the heat exchanger (12).

3. The CPAP (Continuous Positive Air Pressure) respiratory apparatus of claim 1 or 2 characterized in that the heat exchanger (12) comprises first and second cooling profiles (22; 25; 24).

4. The CPAP (Continuous Positive Air Pressure) respiratory apparatus of claim 3 characterized in that the cold side (21) of the Peltier element (20) is connected to the first cooling profiles (22, 25) and the hot side (23) is connected with the second cooling profile (24).

5. The CPAP (Continuous Positive Air Pressure) respiratory apparatus of claim 3 characterized in that the first cooling profiles (22, 25) have an "E" shape and form a meandering cooling channel for the air stream supplied by the fan (10) between an air entrance (27) and an air exit (28).

6. The CPAP (Continuous Positive Air Pressure) respiratory apparatus of any of the preceding claims characterized in that the supplied air stream is cooled by 2 to 4° C below the ambient temperature.

7. The CPAP (Continuous Positive Air Pressure) respiratory apparatus of any of the preceding claims characterized in that the supplied air stream is cooled by 3° C below the ambient temperature.

## Revendications

1. Dispositif de ventilation à pression d'air positive continue (CPAP) pour, à domicile, ventiler en surpression des personnes présentant le syndrome d'apnée du sommeil obstructive, comprenant une soufflante (10) pour réaliser la surpression et un échangeur thermique (12) disposé dans le flux d'air de la soufflante (10), qui refroidit le flux d'air fourni par la soufflante (10) en dessous de la température ambiante, avant que ce flux ne soit fourni à un masque nasal (14) et à des ouvertures de sortie (15).

2. Dispositif de ventilation à pression d'air positive continue (CPAP) selon la revendication 1, caractérisé en ce que l'échangeur thermique (12) comprend un élément Peltier pour réaliser le refroidissement.

3. Dispositif de ventilation à pression d'air positive continue (CPAP) selon la revendication 1 ou 2, caractérisé en ce que l'échangeur thermique (12) comporte des premier et deuxième profilés de refroidissement (22, 25 ; 24).

4. Dispositif de ventilation à pression d'air positive continue (CPAP) selon la revendication 3, caractérisé en ce que le côté froid (21) de l'élément Peltier (20) est relié à des premiers profilés de refroidissement (22, 25) et le côté chaud (23) est relié au deuxième profilé de refroidissement (24).

5. Dispositif de ventilation à pression d'air positive continue (CPAP) selon la revendication 3, caractérisé en ce que les premiers profilés de refroidissement (22, 25) sont en forme de E et constituent entre une entrée d'air (27) et une sortie d'air (28) un canal de refroidissement en méandre pour le flux d'air fourni par la soufflante (10).

6. Dispositif de ventilation à pression d'air positive continue (CPAP) selon une quelconque des revendications précédentes, caractérisé en ce que le flux d'air fourni est refroidi de 2 à 4°C en dessous de la temperature ambiante.

7. Dispositif de ventilation à pression d'air positive continue (CPAP) selon une quelconque des revendications précédentes, caractérisé en ce que le flux d'air fourni est refroidi de 3°C en dessous de la température ambiante.
